# EUROPEAN PATENT APPLICATION

(11) **EP 2 308 512 A1**
(43) Date of publication of application: **13.04.2011**
(21) Application number: 09773504.7
(22) Date of filing: 01.07.2009
(51) Int. Cl.: A61K 47/34, A61K 8/368, A61K 8/44, A61K 8/49, A61K 8/67, A61K 8/84, A61K 9/08, A61K 31/195, A61K 31/375, A61K 31/60, A61K 47/22, A61P 17/16, A61Q 17/04, A61Q 19/00

(54) **WATER-CONTAINING COMPOSITION**

(30) Priority: 02.07.2008 JP 2008172922; 02.07.2008 JP 2008172924
(71) Applicant: Shiseido Company, Ltd., Chuo-ku Tokyo 104-8010 (JP)
(72) Inventor: TESHIGAWARA Takashi, Yokohama-shi Kanagawa 224-8558 (JP); MIYAHARA Reiji, Yokohama-shi Kanagawa 236-8643 (JP); OCHIAI Masatoshi, Yokohama-shi Kanagawa 224-8558 (JP); OKA Takashi, Yokohama-shi Kanagawa 224-8558 (JP)
(74) Representative: Schön, Christoph
(86) International application number: PCT/JP2009/062036
(87) International publication number: WO 2010/001926

(57) **Abstract**

When a poorly water-soluble UV-absorbing agent is used, difficulty is encountered in solubilization of a sufficient amount of the poorly water-soluble UV-absorbing agent by means of a surfactant such as polyoxyethylene alkyl ether or polyoxyethylene hydrogenated castor oil. An object of the present invention is to provide a water-containing composition in which a poorly water-soluble UV-absorbing agent is solubilized or dispersed in water. Another object of the invention is to stabilize a water-soluble ingredient; i.e., a water-soluble drug or a water-soluble dye, through co-presence of the water-soluble ingredient with the poorly water-soluble UV-absorbing agent dissolved in water. The present inventors have found that the aforementioned objects can be attained through provision of a water-containing composition which contains the following ingredients (1) to (3) and may contain a water-soluble drug and/or a water-soluble dye: (1) a polyoxyethylene addition compound represented by formula HO(CH₂CH₂O₎ₙ-R (I), wherein R represents a phytosterol residue or a phytostanol residue, and n is a number of 5 to 100, (2) a poorly water-soluble UV-absorbing agent, and (3) water.

## Description

### Technical Field

The present invention relates to a water-containing composition which can be employed as a cosmetic composition, a skin agent for external use (hereinafter referred to as external skin agent), etc. The water-containing composition may be such a composition in which a water-soluble drug or water-soluble dye incorporated therein is stabilized.

### Background Art

Many UV-absorbing agents having low water solubility (poorly water-soluble UV-absorbing agents) are useful for external skin products such as cosmetic compositions and external skin agents, and a variety of techniques have been provided for incorporating such a UV-absorbing agent in a stable state into an external-use composition. Among such techniques, emulsification or solubilization is typically employed. For the purpose of delivering a UV-absorbing agent to the skin with light feeling, solubilization or water-dispersion is employed. In particular, a solubilization technique is essentially employed, when the UV-absorbing agent is incorporated into a skin lotion or the like. Generally, a surfactant such as polyoxyethylene alkyl ether or polyoxyethylene hydrogenated castor oil is employed as a solubilizing agent for the UV-absorbing agent.

Patent Document 1 discloses use of ethoxylated phytosterol and phytostanol as means for dissolving a poorly water-soluble pharmaceutical agent. However, Patent Document 1 realize manufacture of an aqueous solution of the pharmaceutical agent for prevention of cell damage of living cells, and Patent Document 1 fails to disclose an UV-absorbing agent or use thereof.

The UV-absorbing agent may be used as an agent for protecting the skin against UV rays and also as an agent for stabilizing a water-soluble dye or a water-soluble drug. Water-soluble dyes are mainly employed as coloring agents in external skin products such as cosmetic products and external agents. These water-soluble dyes are known to be degraded and undergo color fading upon exposure to UV rays included in sunlight or other light sources. Similarly, many water-soluble drugs (usually employed as active ingredients in pharmaceutical products) are also known to undergo promoted degradation and denaturation upon exposure to UV rays. Under such circumstances, a variety of techniques have been provided for incorporating a water-soluble dye or drug in a stable state. One known technique is incorporation of the aforementioned ingredient to be stabilized and an UV-absorbing agent.

Patent Document 2 discloses use of a water-soluble UV-absorbing agent as means for stabilizing a cosmetic composition in which an L-ascorbic acid derivative has been dissolved. However, Patent Document 2 fails to disclose incorporation of a highly effective oil-soluble UV-absorbing agent into the composition, and color fading of a water-soluble dye.

### Prior Art Documents

### Patent Documents

Patent Document 1: Japanese Patent Application Laid-Open *(kokai)* No. 2005-511586
Patent Document 2: Japanese Patent Application Laid-Open *(kokai)* No. Hei 6-172153

### Summary of the Invention

### Problems to be Solved by the Invention

Under such circumstances, when a poorly water-soluble UV-absorbing agent having a large molecular weight and particularly a bulky structure is used, great difficulty is thought to be encountered in solubilization of a sufficient amount of the poorly water-soluble UV-absorbing agent by means of a surfactant such as polyoxyethylene alkyl ether or polyoxyethylene hydrogenated castor oil. Thus, such a technical problem involved in solubilization becomes a technical problem involved in stabilization of a water-soluble ingredient, since the UV-absorbing agent, which exhibits excellent stabilization effect on a water-soluble dye or a water-soluble drug, has poor water solubility.

An object of the present invention is to provide a water-containing composition in which a poorly water-soluble UV-absorbing agent is solubilized or dispersed in water. Another object of the invention is to stabilize a water-soluble ingredient; i.e., a water-soluble drug or a water-soluble dye, through co-presence of the water-soluble ingredient with the poorly water-soluble UV-absorbing agent dissolved in water.

### Means for Solving the Problems

In order to attain the aforementioned objects, the present inventors have conducted extensive studies on surface active substances which can dissolve a poorly water-soluble UV-absorbing agent in water, and have found that the poorly water-soluble UV-absorbing agent can be dissolved in an aqueous system through co-presence of the UV-absorbing agent with polyoxyethylene-added phytosterol or phytostanol. The present invention has been accomplished on the basis of this finding.

Accordingly, the present invention provides a water-containing composition comprising the following ingredients: (1) to (3) (hereinafter may be referred to as "water-containing composition of the present invention"):
(1) a polyoxyethylene addition compound represented by formula (I) (hereinafter the compound may be referred to as "compound (I)"):

   HO(CH₂CH₂O)ₙ-R (I),

   wherein R represents a phytosterol residue or a phytostanol residue, and n is a number of 5 to 100;
(2) a poorly water-soluble UV-absorbing agent; and
(3) water.

In addition, the present inventors have conducted studies on stabilization of a drug and a dye in water in which the poorly water-soluble UV-absorbing agent has been dissolved, and have found that the poorly water-soluble UV-absorbing agent can be dissolved in an aqueous system with the compound (I) and a water-soluble drug or a water-soluble dye, to thereby sufficiently stabilize the water-soluble ingredient, and have accomplished the present invention.

Accordingly, the present invention also provides a water-containing composition comprising a water-soluble drug and/or a water-soluble dye, and the aforementioned ingredients (1) to (3) contained in the water-containing composition of the present invention (hereinafter the composition may be referred to as "specific-ingredient-stabilized composition of the present invention"). The invention also provides a method for stabilizing a water-soluble ingredient in a water-containing composition, the method comprising causing a water-soluble drug and/or a water-soluble dye to be co-present with the ingredients (1) to (3) in the water-containing composition, to thereby enhance stability of the drug and/or dye, wherein the ratio by mass of the poorly water-soluble UV-absorbing agent to compound (I) is 1 or higher (hereinafter the method may be referred to as "method for stabilizing a water-soluble ingredient of the present invention").

Compound (I) may be represented by the following formula.

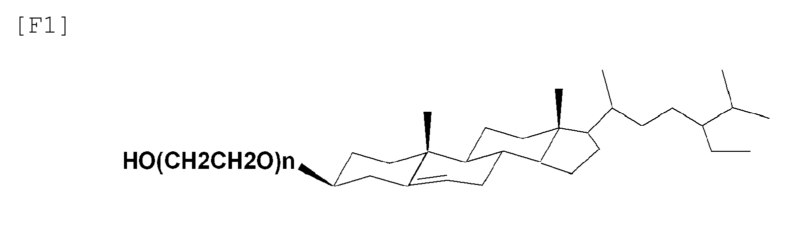

The group to the right of the polyoxyethylene chain in the chemical formula represents a phytosterol residue or a phytostanol residue, represented by R above.

As used herein, the term "water-containing composition" refers to a composition containing water. As used herein, the term "dissolved state" refers to the case where, when the water-containing composition is visually observed under no influence of other ingredients, the UV-absorbing agent is found to be uniformly present in a transparent or semi-transparent state in the system. The "transparent or semi-transparent state" may be, for example, a state where a poorly water-soluble UV-absorbing agent is thermodynamically stable (i.e., "solubilized state"), or a state where the UV-absorbing agent is dispersed in the form of fine particles in the aqueous phase (i.e., "dispersion state in water").

Typically, the water-containing composition of the present invention may be employed as an external-use composition that can be applied to the skin, or may be employed as a base of an external-use composition. Specific product forms of the external-use composition include a cosmetic composition and an external skin agent. Both the cosmetic composition and the external skin agent are in a form that can be applied to the skin, and the concepts of these words mutually overlap. In particular, when a composition is a cosmetic composition, the composition is also an external skin agent. The case where a composition is not a cosmetic composition but an external skin agent corresponds to a case where the composition is not a "cosmetic" for the primary purpose of beauty, but a "quasi-drug" or "drug" for the primary purpose of healthcare.

### Effects of the Invention

The present invention enables provision of (1) a water-containing composition in which a poorly water-soluble UV-absorbing agent is solubilized or dispersed in water (the water-containing composition of the present invention), (2) a water-containing composition comprising a water-soluble drug and/or a water-soluble dye in a stabilized state (the specific-ingredient-stabilized composition of the present invention), and (3) a method for stabilizing the water-soluble drug and/or the water-soluble dye in the water-containing composition of the present invention (the stabilizing method of the present invention).

### Modes for Carrying Out the Invention

### [A] The water-containing composition of the present invention

### [A-1] Incorporation of polyoxyethylene addition compound (compound (I))

As described above, the water-containing composition of the present invention contains compound (I) together with water and a poorly water-soluble UV-absorbing agent. As described hereinbelow, compound (I) contained in the water-containing composition of the present invention may be only one compound represented by formula (I), or a mixture of two or more different compounds represented by formula (I).

No particular limitation is imposed on the phytosterol, which is a compound that provides a phytosterol residue represented by R in compound (I), and is a plant-derived sterol (F. D. Gunstone and B. G. Herslof, A Lipid Glossary, The Oily Press, Air, 1992). Examples of the phytosterol residue represented by R include a sitosterol residue, a campesterol residue, a stigmasterol residue, a brassicasterol residue, an avenasterol residue, and an ergosterol residue. As described above, compound (I) contained in the water-containing composition of the present invention may be a mixture of two or more compounds having different phytosterol residues represented by R.

Similarly, no particular limitation is imposed on the phytostanol, which is a compound that provides a phytostanol residue represented by R in compound (I), and is obtained through hydrogenation or saturation of the corresponding phytosterol. Examples of the phytostanol residue represented by R include sitostanol, campestanol, stigmastanol, brassicastanol, avenastanol, and ergostanol. As described above, compound (I) contained in the water-containing composition of the present invention may be a mixture of two or more compounds having different phytostanol residues represented by R.

Compound (I) contained in the water-containing composition of the present invention may be a mixture of one compound having a phytosterol residue represented by R, or two or more compound having different phytosterol residues, and one compound having a phytostanol residue represented by R, or two or more compound having different phytostanol residues.

In compound (I), n, which represents the number of the polyoxyethylene chains, is 5 to 100, preferably 5 to 50.

Compound (I) may be produced through a customary method, on the basis of the chemical structure thereof. For example, compound (I) may be readily produced through the following procedure: ethylene oxide is added to phytosterol in the presence of a catalyst; unreacted matter is removed; and the product is subjected to neutralization, dehydration, deodorization, and filtration.

The amount of compound (I) contained in the water-containing composition of the present invention is 10 mass% or less, preferably 3 mass% or less, on the basis of the entirety of the composition. The amount of compound (I) greatly depends on the relationship between the amount thereof and that of the poorly water-soluble UV-absorbing agent incorporated into the composition. The ratio by mass of compound (I) to the poorly water-soluble UV-absorbing agent in the composition is preferably 1 or higher, particularly preferably 3 or higher (the upper limit of this ratio is determined on the basis of the upper limit of the amount of compound (I)). A suitable lower limit of the amount of compound (I) may be determined on the basis of this ratio by mass. Notably, since compound (I) is an essential ingredient of the water-containing composition of the present invention, the amount of compound (I) is not 0 mass%.

### [A-2] Incorporation of poorly water-soluble UV-absorbing agent

Into the water-containing composition of the present invention, one or more poorly water-soluble UV-absorbing agents are incorporated, and these agents are solubilized or dispersed in water. No particular limitation is imposed on the solubility of the poorly water-soluble UV-absorbing agent in a solvent other than water. Incorporation of the poorly water-soluble UV-absorbing agent does not exclude optional use of a water-soluble UV-absorbing agent.

Examples of the poorly water-soluble UV-absorbing agent include triazine UV-absorbing agents, typically bisresorcinyltriazine, more specifically, bisethylhexyloxyphenol methoxyphenyltriazine (2,4-bis{[4-(2-ethylhexyloxy)-2-hydroxy]phenyl}-6-(4-methoxyphenyl)-1,3,5-triazine), TINOSORB S (commercial product of Ciba Specialty Chemicals), octyl triazone (2,4,6-tris[4-(2-ethylhexyloxycarbonyl)anilino]-1,3,5-triazine), 2,4,6-trianilino-p-(carbo-2'-ethylhexyl-1'-oxy)-1,3,5-triazine, UVINUL T150 (commercial product of BASF), and 2-[2-hydroxy-4-(2-ethylhexyl)phenoxy]-2H-benzotriazole; benzoic acid UV-absorbing agents (e.g., p-aminobenzoic acid (hereinafter abbreviated as "PABA"), PABA monoglycerin ester, N,N-dipropoxy-PABA ethyl ester, N,N-diethoxy-PABA ethyl ester, N,N-dimethyl-PABA ethyl ester, and N,N-dimethyl-PABA butyl ester); anthranilic acid UV-absorbing agents (e.g., homomenthyl-N-acetyl anthranilate); salicylic acid UV-absorbing agents (e.g., amyl salicylate, menthyl salicylate, homomenthyl salicylate, octyl salicylate, phenyl salicylate, benzyl salicylate, and p-isopropanolphenyl salicylate); cinnamic acid UV-absorbing agents (e.g., octyl cinnamate, ethyl 4-isopropylcinnamate, ethyl 2,4-diisopropylcinnamate, methyl 2,4-diisopropylcinnamate, propyl p-methoxycinnamate, isopropyl p-methoxycinnamate, cyclohexyl p-methoxycinnamate, ethyl a-cyano-β-phenylcinnamate, and 2-ethylhexyl α-cyano-β-phenylcinnamate); benzophenone UV-absorbing agents (e.g., 2-[4-(diethylamino)-2-hydroxybenzoyl]benzoic acid hexyl ester, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone); 3-(4'-methylbenzylidene)-d,1-camphor and 3-benzylidene-d,1-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzaladine; dianisoylmethane; 4-methoxy-4'-t-butyldibenzoylmethane; and 5-(3,3-dimethyl-2-norbornylidene)-3-pentan-2-one; phenyl acrylate UV-absorbing agents (e.g., 2-ethylhexyl-2-cyano-3,3-diphenyl acrylate (octocrylene) and 2-ethyl-2-cyano-3,3-diphenyl acrylate); dibenzoylmethane UV-absorbing agents (e.g., 4-tert-butyl-4'-methoxydibenzoylmethane); camphor derivatives (e.g., 4-methylbenzylidene camphor and terephthalylidene dicamphor sulfonic acid); phenylbenzotriazole derivatives (e.g., hydroxy-(ethylhexyl)phenoxybenzotriazole and methylenebis-benzotriazolyltetramethylbutylphenol); and benzalmalonate derivatives (e.g., dimethicone benzalmalonate).

Among the aforementioned poorly water-soluble UV-absorbing agents, a poorly water-soluble UV-absorbing agent having, in the chemical structure thereof, two or more 6-membered rings is preferably employed. Examples of such a UV-absorbing agent include triazine derivatives such as bisethylhexyloxyphenol methoxyphenyltriazine, 2,4,6-tris[4-(2-ethylhexyloxycarbonyl)anilino]-1,3,5-triazine, and 2-[2-hydroxy-4-(2-ethylhexyl)phenoxy]-2H-benzotriazole. Of these, bisethylhexyloxyphenol methoxyphenyltriazine is preferred.

The UV-absorbing agent having an absorption band in the UVA region tends to exhibit an excellent effect of stabilizing a water-soluble dye or a water-soluble drug. Thus, such a UV-absorbing agent is preferably chosen as a poorly water-soluble UV-absorbing agent for producing the specific-ingredient-stabilized composition of the present invention described hereinbelow. Examples of such poorly water-soluble UV-absorbing agents include bisethylhexyloxyphenol methoxyphenyltriazine, 2,4,6-tris[4-(2-ethylhexyloxycarbonyl)anilino]-1,3,5-triazine, 2-[2-hydroxy-4-(2-ethylhexyl)phenoxy]-2H-benzotriazole, 4-tert-butyl-4'-methoxybenzoylmethane, and 2-[4-(diethylamino)-2-hydroxybenzoyl]benzoic acid hexyl ester.

No particular limitation is imposed on the amount of the poorly water-soluble UV-absorbing agent contained in the water-containing composition of the present invention, so long as the poorly water-soluble UV-absorbing agent can be maintained in a solubilized or water-dispersed state in the composition at ambient temperature. The relationship between the amount of the poorly water-soluble UV-absorbing agent and that of compound (I) is as described above. The specific amount of the poorly water-soluble UV-absorbing agent may be determined within the limits of the relationship, in consideration of the property of the UV-absorbing agent to be incorporated.

### [A-3] Incorporation of water

The water employed in the water-containing composition of the present invention is generally ion-exchange water, purified water, or tap water. The amount of water contained in the water-containing composition of the present invention may be the balance of the entire composition containing compound (I) and the poorly water-soluble UV-absorbing agent, or the balance of the entire composition containing compound (I), the poorly water-soluble UV-absorbing agent, and a common ingredient incorporated into the composition.

### [A-4] Specific embodiments of the water-containing composition of the present invention

In the water-containing composition of the present invention, by virtue of the presence of compound (I), the UV-absorbing agent, which is intrinsically difficult to dissolve in water, is readily solubilized or water-dispersed. Preferably, the water-containing composition of the present invention is produced by, for example, dissolving or dispersing a poorly water-soluble UV-absorbing agent in compound (I) to thereby prepare a portion, and mixing the portion with an aqueous phase for solubilization or dispersion of the UV-absorbing agent.

One embodiment of the water-containing composition of the present invention containing only the aforementioned essential ingredients may be provided as an external-use composition used in the form of cosmetic composition or external skin agent. The external-use composition may cause the effects (e.g., chemical effect and UV-shielding action) of the solubilized or water-dispersed poorly soluble UV-absorbing agent on the skin.

The above embodiment of the water-containing composition of the present invention containing only the aforementioned essential ingredients may also be employed as a base for preparing an external-use composition into which a common ingredient other than the essential ingredients is incorporated. Specifically, a final external-use composition of interest may be produced by firstly producing a basic water-containing composition of the present invention containing only the aforementioned essential ingredients, and secondly incorporating an optional ingredient into the composition. The thus-produced external-use composition is also an embodiment of the water-containing composition of the present invention.

Such a common ingredient is selected in consideration of, for example, provision of medicinal effects, coloration, regulation of production parameters, adjustment of ionic strength, adjustment of pH, or stability of a product. Examples of the common ingredient include an excipient, a buffer, a salt, an antioxidant, a preservative, a perfume, a surfactant, and a water-soluble vitamin. The common ingredient employed is generally a water-soluble substance. However, the common ingredient may be an oil-soluble ingredient, depending on the form of a final product. For example, a liquid oil ingredient having no 6-membered ring in the structure may be employed as such a common ingredient.

The final target product of the water-containing composition of the present invention may also be produced without producing the embodiment of the water-containing composition of the present invention having a basic formulation as described above. In such a case, the final product of the water-containing composition of the present invention may be produced through, for example, the following process: a water-soluble ingredient selected as a common ingredient is dissolved in water to thereby prepare an aqueous phase; and the aqueous phase is mixed with a portion prepared by dissolving or dispersing a poorly water-soluble UV-absorbing agent in compound (I), to thereby solubilize or disperse the poorly water-soluble UV-absorbing agent in water. However, the method for producing the final composition of interest is not limited to this production process, and may be appropriately selected or devised according to need or in consideration of the property of the selected common ingredient.

### [B] Specific-ingredient-stabilized composition of the present invention

### [B-1] Ingredients also contained in the water-containing composition of the present invention

The specific-ingredient-stabilized composition of the present invention is a specific embodiment of the aforementioned water-containing composition of the present invention, which contains one or more water-soluble drugs and/or one or more water-soluble dyes. Thus, (1) compound (I), (2) a poorly water-soluble UV-absorbing agent, and (3) water, which are incorporated as essential ingredients into the specific-ingredient-stabilized composition of the present invention , are the same as described in (A-1), (A-2), and (A-3) of the specific-ingredient-stabilized composition of the present invention.

### [B-2] Ingredients stabilized in the specific-ingredient-stabilized composition

### (1) Water-soluble dye

No particular limitation is imposed on the water-soluble dye to be stabilized in the specific-ingredient-stabilized composition of the present invention. Examples of the dye include Red No. 3 (Erythrocin), Red No. 102 (New Coccine), Red No. 106 (Acid Red), Red No. 201 (Lithol Rubin B), Red No. 227 (Fast Acid Magenta), Red No. 230(1) (Erythrocin YS), Red No. 203(2) (Erythrocin YSK), Red No. 231 (Phloxine BK), Red No. 232 (Rose Bengal K), Red No. 401 (Violamine R), Red No. 502 (Ponceau 3R), Red No. 503 (Ponceau R), Red No. 504 (Ponceau SX), Red No. 506 (Fast Red S), Yellow No. 202(2) (Uranine K), Yellow No. 4 (Tartrazine), Yellow No. 402 (Polar Yellow 5G), Yellow No. 403(1) (Naphthol Yellow S), Yellow No. 406 (Metanil Yellow), Green No. 3 (Fast Green FCF), Green No. 201 (Alizarine Cyanine Green F), Green No. 204 (Pyranine Conc), Green No. 205 (Light Green SF Yellow), Green No. 401 (Naphthol Green B), Green No. 402 (Guinea Green B), Blue No. 1 (Brilliant Blue FCF), Blue No. 2 (Indigo Carmine), Blue No. 202 (Patent Blue NA), Blue No. 205 (Alphazurine FG), Brown 201 (Resorcin Brown), Purple 401 (Alizurol Purple), and Black 401 (Naphthol Blue Black).

### (2) Water-soluble drug

No particular limitation is imposed on the water-soluble drug to be stabilized in the specific-ingredient-stabilized composition of the present invention. Examples of the drug include, as a skin-whitening agent, L-ascorbic acid (vitamin C), vitamin C derivatives such as ascorbic acid inorganic salt esters (e.g., L-ascorbyl-monophosphate, L-ascorbic acid 2-sulfate, and L-ascorbic acid dl-a-tocopherol phosphoric acid diester); and ascorbic acid 2-glucosides such as 2-O-α-D-glucopyranosyl-L-ascorbic acid.

These ascorbic acid and derivatives thereof are generally incorporated into the composition in the salt form. Examples of the salt include alkali metal salts (e.g., Na salts and K salts), alkaline earth metal salts (e.g., Ca salts and Mg salts), ammonium salts, alkanolamine salts, and amino acid salts. Of these, alkali metal salts are preferred.

Examples of the substance to be stabilized in the specific-ingredient-stabilized composition of the present invention also include salicylic acid or a salicylic acid derivative. Examples of an alkloxysalicylic acid (salicylic acid derivative) skin-whitening agent include those disclosed in Japanese Patent Application Laid-Open (*kokai*) No. Hei 6-40886. Specific examples include 3-methoxysalicylic acid, 3-ethoxysalicylic acid, 4-methoxysalicylic acid, 4-ethoxysalicylic acid, 4-propoxysalicylic acid, 4-isopropoxysalicylic acid, 4-butoxysalicylic acid, 5-methoxysalicylic acid, 5-ethoxysalicylic acid, 5-propoxysalicylic acid, and salts thereof. Examples of such salts include alkali metal salts (e.g., Na salts and K salts), alkaline earth metal salts (e.g., Ca salts and Mg salts), ammonium salts, alkanolamine salts, and amino acid salts. Of these, alkali metal salts are preferred.

Furthermore, tranexamic acid or a tranexamic acid derivative may also be incorporated as a water-soluble drug into the specific-ingredient-stabilized composition of the present invention. Examples of the tranexamic acid derivative include tranexamic acid dimers (e.g., trans-4-(transaminomethylcyclohexanecarbonyl)aminomethylcyclohexaneca rboxylic acid hydrochloride); tranexamic acid hydroquinone esters (e.g., trans-4-aminomethylcyclohexanecarboxylic acid 4'-hydroxyphenyl ester); tranexamic acid gentisic acid esters (e.g., 2-(trans-4-aminomethylcyclohexanecarbonyloxy)-5-hydroxybenzoic acid and a salt thereof); and tranexamic acid amides (e.g., trans-4-aminomethylcyclohexanecarboxylic acid methylamide and a salt thereof, trans-4-(p-methoxybenzoyl)aminomethylcyclohexanecarboxylic acid and a salt thereof, and trans-4-guanidinoaminomethylcyclohexanecarboxylic acid and a salt thereof).

Examples of other water-soluble drugs which can be incorporated into the specific-ingredient-stabilized composition of the present invention include amino acids such as glycine, alanine, valine, leucine, threonine, phenylalanine, tyrosine, aspartic acid, asparagine, glutamine, taurine, arginine, and histidine, and alkali metal salts and hydrochlorides thereof; acylsarcosinic acid (e.g., sodium laurolysarcosinate) and glutathione; organic acids such as citric acid, malic acid, tartaric acid, and lactic acid; vitamin A and its derivatives; vitamin Bs such as vitamin B₆ hydrochloride, vitamin B₆ tripalmitate, vitamin B₆ dioctanoate, vitamin B₂ and its derivatives, vitamin B₁₂, vitamin B₁₅ and its derivatives; vitamin Es; vitamin Ds; other vitamins such as vitamin H, pantothenic acid, and pantethine; nicotinamide, benzyl nicotinate, γ-oryzanol, allantoin, glycyrrhizinic acid (salt), glycyrrhetinic acid and its derivatives, hinokitiol, bisabolol, eucaryptol, thymol, and inositol; saponins such as saiko-saponin, ginseng saponin, luffa cylindrica saponin, soapberry saponin; and other drugs such as pantothenyl ethylether, ethinylestradiol, arbutin, cepharanthine, and placenta extract.

No particular limitation is imposed on other water-soluble drugs which can be incorporated into the specific-ingredient-stabilized composition of the present invention, so long as the drugs are generally employed in cosmetic products. Examples of such water-soluble drugs include angelica keiskei extract, gambir extract, althea officinalis extract, arnica montana extract, aloe extract, aloe vera extract, ginkgo biloba extract, nettle extract, fennel extract, rosa multiflora extract, isodonis japonicus extract, scutellaria baicalensis extract, phellodendoron amurense extract, coptis japonica extract, white nettle extract, watercress extract, seaweed extract, mugwort extract, seaweed extract, matricaria extract, avena sativa extract, artemisia capillaris extract, gardenia florida extract, sasa veitchii extract, sophora angustifolia extract, clematis vitalba extract, geranium thunbergii extract, camellia sinensis extract, burdock extract, rice bran extract, comfrey extract, cactus extract, sage extract, crataegus cuneata fruit extract, rehmannia root extract, perilla extract, meadowsweet extract, peony root extract, houttuynia cordata extract, ginger extract, calamus extract, betula alba extract, water-soluble lithospermum officinate extract, ivy extract, yarrow extract, peppermint extract, linden extract, mallow extract, swertia japonica extract, morus alba extract, soybean extract, thyme extract, thymus vulgaris extract, camellia sinensis extract, clove extract, citrus unshiu peel extract, capsicum frutescens extract, Japanese angelica extract, calendula officinalis extract, bitter orange peel extract, ginseng extract, dog rose hips extract, parsley extract, witch hazel extract, rose extract, eriobotrya japonica leaf extract, grape leaf extract, luffa cylindrica extract, safflower extract, typha latifolia extract, paeonia suffruticosa extract, hops extract, quince seed extract, horse chestnut extract, rosemary extract, balm mint extract, sweet clover extract, peach leaf extract, cornflower extract, saxifraga sarmentosa extract, eucalyptus extract, lily extract, Job's tears extract, lavender extract, lemon extract, rosemary extract, chamomile extract, sanguisorba officinalis extract, kiwi fruit extract, grapefruit extract, and royal jelly extract.

Among the above-listed water-soluble drugs, vitamin C, the aforementioned vitamin C derivatives, salicylic acid, the aforementioned salicylic acid derivatives, tranexamic acid, and the aforementioned tranexamic acid derivatives are particularly preferred drugs to be incorporated into the specific-ingredient-stabilized composition of the present invention.

In the case where a water-soluble drug which forms a salt is employed as an ingredient to be stabilized in the specific-ingredient-stabilized composition of the present invention, the water-soluble drug in the salt form may be incorporated. Alternatively, the free-form water-soluble drug in the composition may be neutralized with an alkali agent. No particular limitation is imposed on the alkali agent for neutralizing the water-soluble drug to form its salt, so long as the alkali agent can form a salt of interest. Examples of the alkali agent include metal hydroxides such as sodium hydroxide and potassium hydroxide; alkanolamines such as monoethanolamine, diethanolamine, and triethanolamine; organic acid salts such as sodium citrate, potassium malate, and sodium lactate; and amino acids such as lysine. Of these, alkali metal hydroxides such as sodium hydroxide and potassium hydroxide are preferred.

### [B-3] Specific embodiments of the specific-ingredient-stabilized composition of the present invention

In the specific-ingredient-stabilized composition of the present invention, by virtue of the presence of compound (I), the UV-absorbing agent, which is intrinsically difficult to dissolve in water, is readily solubilized or water-dispersed. Preferably, the specific-ingredient-stabilized composition of the present invention is produced by, for example, dissolving or dispersing a poorly water-soluble UV-absorbing agent in compound (I) to thereby prepare a portion, and mixing the portion with an aqueous phase containing a water-soluble drug or dye to be stabilized, for solubilizaion or dispersion of the UV-absorbing agent.

One embodiment of the specific-ingredient-stabilized composition of the present invention containing only the aforementioned essential ingredients may be provided as an external-use composition used in the form of cosmetic composition or external skin agent. The external-use composition may cause the effects of the stabilized water-soluble dye or drug as well as the effects (e.g., chemical effect and UV-shielding action) of the solubilized or water-dispersed poorly soluble UV-absorbing agent on the skin (including scalp and hair).

The above embodiment of the specific-ingredient-stabilized composition of the present invention containing only the aforementioned essential ingredients may also be employed as a base for preparing an external-use composition into which a common ingredient other than the essential ingredients is incorporated. Specifically, a final external-use composition of interest may be produced by firstly producing a basic specific-ingredient stabilized composition of the present invention containing only the aforementioned essential ingredients, and secondly incorporating an optional ingredient into the composition. The thus-produced external-use composition is also an embodiment of the specific-ingredient-stabilized composition of the present invention.

Such a common ingredient is selected in consideration of, for example, regulation of production parameters, adjustment of ionic strength, adjustment of pH, or stability of a product. Examples of the common ingredient include an excipient, a buffer, a salt, an antioxidant, a preservative, a perfume, a surfactant, and a water-soluble vitamin. The common ingredient employed is generally a water-soluble substance. However, the common ingredient may be an oil-soluble ingredient, depending on the form of a final product. For example, a liquid oil ingredient having no 6-membered ring in the structure may be employed as such a common ingredient.

The final target product of the specific-ingredient-stabilized composition of the present invention may also be produced without producing the embodiment of the specific-ingredient-stabilized composition of the present invention having a basic formulation as described above. In such a case, the final product of the specific-ingredient-stabilized composition of the present invention may be produced through, for example, the following process: the aforementioned water-soluble dye or drug and a water-soluble ingredient selected as a common ingredient are dissolved in water to thereby prepare an aqueous phase; and the aqueous phase is mixed with a portion prepared by dissolving or dispersing a poorly water-soluble UV-absorbing agent in compound (I), to thereby solubilize or disperse the poorly water-soluble UV-absorbing agent in water. However, the method for producing the final composition of interest is not limited to this production process, and may be appropriately selected or devised according to need or in consideration of the property of the selected common ingredient.

### [B-4] Stabilization method of the present invention

As described above, the stabilization method of the present invention is substantially equivalent to a method for stabilizing a water-soluble dye and/or a water-soluble drug contained in the specific-ingredient-stabilized composition of the present invention. Therefore, working of the specific-ingredient-stabilized composition of the present invention leads to carrying out the stabilization method of the present invention.

### Examples

The present invention will next be described in detail by way of Examples, which should not be construed as limiting the invention thereto. Unless otherwise specified, the amount of ingredient is based on mass% with respect to the object into which the ingredient is incorporated. The Examples include those with respect to the water-containing composition of the present invention (group A) and those with respect to the specific-ingredient-stabilized composition (group B).

### [A] Examples with respect to water-containing composition

### [Test Examples A]

A UV-absorbing agent was mixed with and dissolved in compound (1) or another surfactant through a customary method. The resultant portion was mixed with water, to thereby prepare a test sample shown in Tables A1 to A6 (Examples or Comparative Examples). The sample was evaluated according to the below-described criteria. The results of samples of each test system are shown in the corresponding table. Throughout the groups A and B, in each table, blanks (-) in rows corresponding to the amounts of ingredients incorporated represent "0 mass%." "POE" refers to "polyoxyethylene," and "POP" refers to "polyoxypropylene."

### (A-1) Test immediately after preparation of test sample

### (a) Evaluation of dissolved state

The dissolved state of each test sample was visually evaluated according to the following criteria:

○○: transparent;
○: generally transparent;
△: turbid to semi-transparent; and
X: phase separation observed.

### (b) Evaluation of transparency (L value)

The transparency of each test sample was evaluated through measurement of L value. L value was determined by means of a spectrophotometer (UV-160, product of Shimadzu Corporation). The transparency (L value) of each test sample was evaluated on the basis of the transparency of distilled water as a control (L value = 100) according to the following criteria:

≥90: transparent;
≥70 & <90: generally transparent;
<70: turbid to semi-transparent; and
not determined: phase separation observed.

### (c) Sensation test upon use

The sensation upon use of each test sample immediately after preparation was evaluated by 10 expert panelists according to the following criteria:

○: 5 or more of the 10 expert panelists assessed that the test sample exhibited no stickiness;
△: 3 to 4 of the 10 expert panelists assessed that the test sample exhibited no stickiness; and
X: 0 to 2 of the 10 expert panelists assessed that the test sample exhibited no stickiness.

### (A-2) Test on change over time

### (a) Checking of change in dissolution state

Each of the above-prepared test samples was stored at 50°C for one month (hereinafter represented by "50°C1M"), and the L value of the thus-stored sample was measured in a manner similar to that described above. The stability over time of each test sample was evaluated on the basis of the difference between the L value determined immediately after preparation and the L value determined after one-month storage. The following criteria were used for evaluation. The L values determined after one-month storage are shown in the corresponding tables.

○○: difference in L value ≤±2;
○: L value ≤±5;
△: L value ≤±10; and
X: difference in L value ≥±10, or phase separation observed.

<Test system A1>

**[Table A1]**

| | Comp. Ex. A1 | Comp. Ex. A2 | Ex. A1 | Ex. A2 | Ex. A3 |
|---|---|---|---|---|---|
| Ion-exchange water | to 100 | to 100 | to 100 | to 100 | to 100 |
| POE hydrogenated castor oil | 0.5 | - | - | - | - |
| POE-POP-added decyl tetradecyl ether | - | 0.5 | - | - | - |
| POE(30) phytosterol | - | - | 0.5 | 0.5 | 1.0 |
| Bisethylhexyloxyphenolmethoxy phenyltriazine^{*1} | 0.1 | 0.1 | 0.1 | - | 0.5 |
| 2,4,6-Tris[4-(2-ethylhexyloxycarbonyl) anilino]-1,3,5-triazine^{*2} | - | - | - | 0.1 | - |
| Dissolution state (immediately after preparation) | X | X | ○○ | ○○ | ○○ |
| L value (immediately after preparation) | 15 | 12 | 95 | 96 | 99 |
| Change over time in dissolution state (after 50°C1M) | X (separation) | X (separation) | ○○ | ○○ | ○○ |
| L value (after 50°C1M) | 12 | 10 | 95 | 96 | 98 |

| | | | | | |
|---|---|---|---|---|---|
| *1: TINOSORB S (Ciba Specialty Chemicals) *2: UVINUL T150 (BASF) | | | | | |

The same being applied throughout the description.

As shown in Table A1, the surfactant contained in the test sample of Comparative Example A1 or A2, which has no phytosteryl skeleton, exhibited poor ability to solubilize a UV-absorbing agent; i.e., the surfactant was found to be unsuitable for solubilization of the UV-absorbing agent. Each of the test samples of Examples A1 to A3 contained compound (I) having a phytosteryl skeleton as a surfactant. Thus, a triazine UV-absorbing agent, which is a poorly water-soluble UV-absorbing agent, was able to be solubilized at a practically sufficient level.

<Test system A2>

**[Table A2]**

| | Comp. Ex. A3 | Ex. A4 | Ex. A5 |
|---|---|---|---|
| Ion-exchange water | to 100 | to 100 | to 100 |
| POE(2) phytosterol | 0.7 | - | - |
| POE(20) phytosterol | - | 0.7 | - |
| POE(70) phytosterol | - | - | 0.7 |
| 2,4,6-Tris[4-(2-ethylhexyloxycarbonyl)anilino] -1,3,5-triazne | 0.1 | 0.1 | 0.1 |
| Dissolution state (immediately after preparation) | ○ | ○○ | ○○ |
| L value (immediately after preparation) | 87 | 92 | 97 |
| Sensation upon use | ○ | ○ | △ |
| Change over time in dissolution state (after 50°C1M) | ○ | ○○ | ○○ |
| L value (after 50°C1M) | 82 | 92 | 97 |

As is clear from Table A2, the test sample of Comparative Example A3, containing polyoxyethylene-added phytosterol having <5 moles of polyoxyethylene groups, exhibited slightly poor stability over time.

The test sample of Example A4, containing compound (I) having 5 to 50 moles of polyoxyethylene groups, exhibited good appearance, sensation upon use, and stability over time.

The test sample of Example A5, containing compound (I) having ≥50 moles of polyoxyethylene groups, exhibited stickiness upon use.

### <Test system A3>

The test system shown in Table A3 focused on the ratio by mass of compound (I) to poorly water-soluble substance. Therefore, the ratio is also shown in the table.

**[Table A3]**

| | Ex. A6 | Ex. A7 | Ex. A8 | Ex. A9 |
|---|---|---|---|---|
| Ion-exchange water | to 100 | to 100 | to 100 | to 100 |
| POE(40) phytosterol | 3.5 | 1.5 | 0.8 | 0.4 |
| 4-tert-Butyl-4'-methoxydibenzoylmethane*1 | 0.5 | 0.5 | 0.4 | 0.8 |
| Compound (I)/UV-absorber ratio | 15/1 | 5/1 | 2/1 | 1/2 |
| Dissolution state (immediately after preparation) | ○○ | ○○ | ○○ | ○ |
| L value (immediately after preparation) | 97 | 98 | 95 | 81 |
| Sensation upon use | ○ | ○ | ○ | ○ |
| Change over time in dissolution state (after 50°C1M) | ○○ | ○○ | ○○ | ○ |
| L value (after 50°C1M) | 97 | 98 | 95 | 75 |

| | | | | |
|---|---|---|---|---|
| *1: Parsol 1789 (DSM Nutrition Japan K.K.) | | | | |

The test sample of Example A9, in which the ratio by mass of compound (I) to UV-absorbing agent is 1 or less, exhibited a slightly semi-transparent state.

The test samples of Examples A6 to A8, in which the ratio by mass of compound (I) to UV-absorbing agent is 1 or higher, exhibited good appearance, sensation upon use, and stability.

<Test system A4>

**[Table A4]**

| | Ex. A10 | Ex. A11 | Ex. A12 | Ex. A13 |
|---|---|---|---|---|
| Ion-exchange water | to 100 | to 100 | to 100 | to 100 |
| POE(25) phytosterol | 0.4 | 0.4 | 0.4 | 0.4 |
| 2-Ethylhexyl p-methoxycinnamate^{*1} | 0.2 | - | - | - |
| Hexyl diethylaminohydroxybenzoylbenzoate^{*2} | - | 0.2 | - | - |
| 2-[2-Hydroxy-4-(2-ethylhexyl)phenoxy]-2H-benzotriazole | - | - | 0.2 | - |
| 2-Ethylhexyl 2-cyano-3,3-diphenylacrylate^{*3} | - | - | - | 0.2 |
| Dissolution state (immediately after preparation) | ○○ | ○○ | ○○ | ○○ |
| L value (immediately after preparation) | 95 | 93 | 96 | 97 |
| Change over time in dissolution state (after 50°C1M) | ○ | ○○ | ○○ | ○○ |
| L value (after 50°C1M) | 91 | 93 | 96 | 97 |

| | | | | |
|---|---|---|---|---|
| *1: Parsol MCX (DSM Nutrition Japan K.K.) *2: UVINUL A+ (BASF) *3: Parsol 340 (DSM Nutrition Japan K.K.) | | | | |

Compound (I) contained in the test sample of Example A10 solubilized a UV-absorbing agent having only one 6-membered ring in the chemical structure thereof, but the test sample failed to achieve best results in terms of stability over time.

The test samples of Example A11 to A13, containing compound (I) and a UV-absorbing agent having two or more 6-membered rings in the chemical structure thereof, exhibited good appearance and stability.

### <Test system A5>

The test system shown in Table A5 employed a formulation (external-use agent) containing common ingredients in addition to essential ingredients of the water-containing composition of the present invention. For preparation of each test sample, the corresponding common ingredients (water-soluble ingredients) were mixed with ion-exchange water to thereby prepare an aqueous phase portion, and subsequently the aqueous phase portion was mixed with a portion prepared by dissolving a UV-absorbing agent in compound (I).

**[Table A5]**

| | Ex. A14 | Ex. A15 | Ex. A16 | Ex. A17 |
|---|---|---|---|---|
| Ion-exchange water | to 100 | to 100 | to 100 | to 100 |
| Ethanol | 5.0 | 5.0 | 5.0 | 5.0 |
| Dynamite glycerin | 3.0 | 3.0 | 3.0 | 3.0 |
| Dipropylene glycol | 2.0 | 2.0 | 2.0 | 2.0 |
| POE(14)-POP(7) dimethyl ether | 1.0 | 1.0 | 1.0 | 1.0 |
| POE(30) phytosterol | 0.4 | 0.4 | 0.4 | 0.4 |
| Citric acid | 0.05 | 0.05 | 0.05 | 0.05 |
| Na citrate | 0.05 | 0.05 | 0.05 | 0.05 |
| Na ascorbate | 2.0 | - | - | - |
| Tranexamic acid | 1.0 | 2.0 | 0.5 | |
| K 4-methoxysalicylate | - | - | 1.0 | 1.0 |
| Phenoxyethanol | 0.5 | 0.5 | 0.5 | 0.5 |
| Perfume | 0.01 | 0.01 | 0.01 | 0.01 |
| Red No. 227 | 0.0001 | 0.0001 | 0.0001 | 0.0001 |
| Bisethylhexyloxyphenolmethoxyphenyl-triazine | 0.1 | 0.1 | 0.1 | 0.1 |
| Dissolution state (immediately after preparation) | ○○ | ○○ | ○○ | ○○ |
| L value (immediately after preparation) | 96 | 96 | 97 | 98 |
| Sensation upon use | ○ | ○ | ○ | ○ |
| Change over time in dissolution state (after 50°C1M) | ○○ | ○○ | ○○ | ○○ |
| L value (after 50°C1M) | 96 | 96 | 97 | 98 |

### [B] Examples with respect to water-containing composition

### [Test Examples B]

A poorly water-soluble UV-absorbing agent was mixed with and dissolved in compound (1) or another surfactant through a customary method. The resultant portion was mixed with a water-soluble dye (Red No. 233), sodium salicylate, potassium 4-methoxysalicylate, tranexamic acid, or sodium ascorbate, serving as an ingredient to be stabilized, in an aqueous medium, to thereby prepare a test sample shown in Tables B1 to B6 (Examples or Comparative Examples). The sample was evaluated according to the below-described criteria.

### (B-1) Test immediately after preparation of test sample

In Test B-1, (a) evaluation of dissolved state, (b) evaluation of transparency (L value), and (c) sensation test upon use were performed. The method and evaluation criteria of Test B-1 were the same as employed in the aforementioned Test A-1; i.e., (a) evaluation of dissolved state, (b) evaluation of transparency (L value), and (c) sensation test upon use.

### (B-2) Test on change over time

### (a) Checking of change in dissolution state

The method and evaluation criteria of Test B-2 were the same as employed in the aforementioned Test A-2; i.e., (a) checking of change in dissolution state.

### (b) Evaluation of color difference (ΔE value)

The color difference (ΔE value) of each test sample was determined by means of a spectrophotometer (UV-160, product of Shimadzu Corporation). The color difference (ΔE value) of each test sample was evaluated by using the sample immediately after preparation as a control according to the following criteria:

ΔE of ≤1: no color change
ΔE of ≤2: virtually no color change
ΔE of ≤5: slight color change
ΔE of ≥5: considerable color change

<Test system B1>

**[Table B1]**

| | Comp. Ex. B1 | Comp. Ex. B2 | Ex. B1 | Ex. B2 | Ex. B3 |
|---|---|---|---|---|---|
| Ion-exchange water | to 100 | to 100 | to 100 | to 100 | to 100 |
| POE hydrogenated castor oil | 0.5 | - | - | - | - |
| POE-POP-added decyl tetradecyl ether | - | 0.5 | - | - | - |
| POE(30) phytosterol | - | - | 0.5 | 0.5 | 1.0 |
| Bisethylhexyloxyphenol-methoxyphenyltriazine | 0.1 | 0.1 | 0.1 | - | 0.5 |
| 2,4,6-Tris[4-(2-ethylhexyloxy-carbonyl)anilino]-1,3,5-triazine | - | - | - | 0.1 | - |
| Na salicylate | 0.1 | 0.1 | 0.1 | 0.1 | 0.1 |
| Red No. 233 | 0.00003 | 0.00003 | 0.00003 | 0.00003 | 0.00003 |
| Dissolution state (immediately after preparation) | X | X | ○○ | ○○ | ○○ |
| L value (immediately after preparation) | 15 | 12 | 95 | 96 | 99 |
| Change over time in dissolution state (after 50°C1M) | X (separation) | X (separation) | ○○ | ○○ | ○○ |
| L value (after 50°C1M) | 12 | 10 | 95 | 96 | 98 |
| ΔE value | 20 | 15 | 1 | 2 | 1 |

As shown in Table B1, the surfactant contained in the test sample of Comparative Example B1 or B2, which has no phytosteryl skeleton, exhibited poor ability to solubilize a poorly water-soluble UV-absorbing agent (bisethylhexyloxyphenolmethoxyphenyltriazine); i.e., the surfactant was found to be unsuitable for solubilization of the UV-absorbing agent. In contrast, since each of the test samples of Examples B1 to B3 contained compound (I) having a phytosteryl skeleton, the aforementioned poorly water-soluble UV-absorbing agent was able to be solubilized at a practically sufficient level.

<Test system B2>

**[Table B2]**

| | Comp. Ex. B3 | Ex. B4 | Comp. Ex. B4 |
|---|---|---|---|
| Ion-exchange water | to 100 | to 100 | to 100 |
| POE(2) phytosterol | 0.7 | - | - |
| POE(20) phytosterol | - | 0.7 | - |
| POE(70) phytosterol | - | - | 0.7 |
| 2,4,6-Tris[4-(2-ethylhexyloxycarbonyl)anilino] -1,3,5-triazine | 0.1 | 0.1 | 0.1 |
| Tranexamic acid | 2.0 | 2.0 | 2.0 |
| Na ascorbate | 1.0 | 1.0 | 1.0 |
| Dissolution state (immediately after preparation) | ○ | ○○ | ○○ |
| L value (immediately after preparation) | 87 | 92 | 97 |
| Sensation upon use | ○ | ○ | △ |
| Change over time in dissolution state (after 50°C1M) | ○ | ○○ | ○○ |
| L value (after 50°C1M) | 82 | 92 | 97 |
| △E value | 7 | 2 | 3 |

As is clear from Table B2, the test sample of Comparative Example B3, containing polyoxyethylene-added phytosterol compound (I) having <5 moles of polyoxyethylene groups, exhibited slightly poor stability over time.

The test sample of Example B4, containing compound (I) having 5 to 50 moles of polyoxyethylene groups, exhibited good appearance, sensation upon use, and stability over time.

The test sample of Comparative Example B4, containing compound (I) having ≥50 moles of polyoxyethylene groups, exhibited stickiness upon use.

### <Test system B3>

The test system shown in Table B3 focused on the ratio by mass of compound (I) to poorly water-soluble substance. Therefore, the ratio is also shown in the table.

**[Table B3]**

| | Ex. B5 | Ex. B6 | Ex. B7 | Ex. B8 |
|---|---|---|---|---|
| Ion-exchange water | to 100 | to 100 | to 100 | to 100 |
| POE(40) phytosterol | 3.5 | 1.5 | 0.8 | 0.4 |
| Bisethylhexyloxyphenolmethoxyphenyltriazine | 0.5 | 0.5 | 0.4 | 0.8 |
| K 4-methoxysalicylate | 1.0 | 1.0 | 1.0 | 1.0 |
| Compound (I)/UV-absorber ratio | 15/1 | 5/1 | 2/1 | 1/2 |
| Dissolution state (immediately after preparation) | ○○ | ○○ | ○○ | ○ |
| L value (immediately after preparation) | 97 | 98 | 95 | 81 |
| Sensation upon use | ○ | ○ | ○ | ○ |
| Change over time in dissolution state (after 50°C1M) | ○○ | ○○ | ○○ | ○ |
| L value (after 50°C1M) | 97 | 98 | 95 | 75 |
| ΔE value | 2 | 3 | 1 | 7 |

The test sample of Example B8, in which the ratio by mass of compound (I) to poorly water-soluble UV-absorbing agent is 1 or less, exhibited a slightly semi-transparent state.

The test samples of Examples B5 to B7, in which the ratio by mass of compound (I) to poorly water-soluble UV-absorbing agent is 1 or higher, exhibited good appearance, sensation upon use, and stability.

<Test system B4>

**[Table B4]**

| | Ex. B9 | Ex. B10 | Ex. B11 | Ex. B12 |
|---|---|---|---|---|
| Ion-exchange water | to 100 | to 100 | to 100 | to 100 |
| POE(25) phytosterol | 0.4 | 0.4 | 0.4 | 0.4 |
| 2-Ethylhexyl p-methoxycinnamate*1 | 0.2 | - | - | - |
| 2-Ethylhexyl 2-cyano-3,3-diphenylacrylate^{*2} | - | 0.2 | - | - |
| Hexyl diethylaminohydroxybenzoylbenzoate^{*3} | - | - | 0.2 | - |
| Bisethylhexyloxyphenolmethoxyphenyl-triazine | - | - | - | 0.2 |
| Red No. 233 | 0.00003 | 0.00003 | 0.00003 | 0.00003 |
| Dissolution state (immediately after preparation) | ○○ | ○○ | ○○ | ○○ |
| L value (immediately after preparation) | 95 | 93 | 96 | 97 |
| Sensation upon use | ○ | ○ | ○ | ○ |
| Change over time in dissolution state (after 50°C1M) | ○ | ○○ | ○○ | ○○ |
| L value (after 50°C1M) | 91 | 93 | 96 | 97 |
| ΔE value | 8 | 9 | 1 | 2 |

| | | | | |
|---|---|---|---|---|
| *1:Parsol MCX (DSM Nutrition Japan K.K.) *2:Parsol 340 (DSM Nutrition Japan K.K.) *3: UVINUL A+ (BASF) | | | | |

Compound (I) contained in the test sample of Example B9 solubilized a poorly water-soluble UV-absorbing agent having only one 6-membered ring in the chemical structure thereof, but the test sample failed to achieve best results in terms of stability over time.

The test samples of Example B10 to B12, containing compound (I) and a poorly water-soluble UV-absorbing agent having two or more 6-membered rings in the chemical structure thereof, exhibited good appearance and stability.

### <Test system B5>

The test system shown in Table B5 employed a formulation (external-use agent) containing common ingredients in addition to essential ingredients of the water-containing composition of the present invention. For preparation of each test sample, the corresponding common ingredients (water-soluble ingredients) were mixed with ion-exchange water to thereby prepare an aqueous phase portion, and subsequently the aqueous phase portion was mixed with a portion prepared by dissolving a poorly water-soluble UV-absorbing agent in compound (I).

**[Table B5]**

| | Ex. B13 | Ex. B14 | Ex. B15 | Ex. B16 |
|---|---|---|---|---|
| Ion-exchange water | to 100 | to 100 | to 100 | to 100 |
| Ethanol | 5.0 | 5.0 | 5.0 | 5.0 |
| Dynamite glycerin | 3.0 | 3.0 | 3.0 | 3.0 |
| Dipropylene glycol | 2.0 | 2.0 | 2.0 | 2.0 |
| POE(14)-POP(7) dimethyl ether | 1.0 | 1.0 | 1.0 | 1.0 |
| POE(30) phytosterol | 0.4 | 0.4 | 0.4 | 0.4 |
| Citric acid | 0.05 | 0.05 | 0.05 | 0.05 |
| Na citrate | 0.05 | 0.05 | 0.05 | 0.05 |
| Na ascorbate | 2.0 | - | - | - |
| Tranexamic acid | 1.0 | 2.0 | 0.5 | - |
| K 4-methoxysalicylate | - | - | 1.0 | 1.0 |
| Phenoxyethanol | 0.5 | 0.5 | 0.5 | 0.5 |
| Perfume | 0.01 | 0.01 | 0.01 | 0.01 |
| Red No. 227 | 0.0001 | 0.0001 | 0.0001 | 0.0001 |
| Bisethylhexyloxyphenolmethoxyphenyl -triazine | 0.1 | 0.1 | 0.1 | 0.1 |
| Dissolution state (immediately after preparation) | ○○ | ○○ | ○○ | ○○ |
| L value (immediately after preparation) | 96 | 96 | 97 | 98 |
| Sensation upon use | ○ | ○ | ○ | ○ |
| Change over time in dissolution state (after 50°C1M) | ○○ | ○○ | ○○ | ○○ |
| L value (after 50°C1M) | 96 | 96 | 97 | 98 |
| ΔE value | 2 | 2 | 1 | 3 |

## Claims

1. A water-containing composition comprising the following ingredients: (1) to (3):
(1) a polyoxyethylene addition compound represented by formula (I):
HO(CH₂CH₂O)ₙ-R (I),
wherein R represents a phytosterol residue or a phytostanol residue, and n is a number of 5 to 100;
(2) a poorly water-soluble UV-absorbing agent; and
(3) water.

2. The water-containing composition according to claim 1, wherein the ratio by mass of the poorly water-soluble UV-absorbing agent to the polyoxyethylene addition compound (I) is 1 or higher.

3. The water-containing composition according to claim 1 or 2, wherein the phytosterol residue or the phytostanol residue represented by R in the polyoxyethylene addition compound (I) is one or more members selected from the group consisting of a sitosterol residue, a campesterol residue, a stigmasterol residue, a brassicasterol residue, an avenasterol residue, an ergosterol residue, a sitostanol residue, a campestanol residue, a stigmastanol residue, a brassicastanol residue, an avenastanol residue, and an ergostanol residue.

4. The water-containing composition according to any of claims 1 to 3, wherein the polyoxyethylene addition compound (I) has a number n of 5 to 50.

5. The water-containing composition according to any of claims 1 to 4, wherein the poorly water-soluble UV-absorbing agent has a chemical structure having two or more 6-membered rings.

6. The water-containing composition according to any of claims 1 to 5, wherein the poorly water-soluble UV-absorbing agent has an absorption band in the UVA region.

7. The water-containing composition according to any of claims 1 to 6, wherein the poorly water-soluble UV-absorbing agent is a triazine derivative.

8. The water-containing composition according to any of claims 1 to 7, which is an external-use composition.

9. The water-containing composition according claim 8, wherein the external-use composition is in the form of cosmetic composition or external skin agent.

10. The water-containing composition according to any of claims 1 to 9, which is employed as a base for producing an external-use composition.

11. The water-containing composition according to any of claims 1 to 10, which contains a water-soluble drug and/or a water-soluble dye.

12. The water-containing composition according to claim 11, wherein the water-soluble drug is one or more species selected from the group consisting of vitamin C, a vitamin C derivative, salicylic acid, a salicylic acid derivative, tranexamic acid, and a tranexamic acid derivative.

13. A method for stabilizing a water-soluble ingredient in a water-containing composition, the method comprising causing a water-soluble drug and/or a water-soluble dye to be co-present with the ingredients (1) to (3):
(1) a polyoxyethylene addition compound represented by formula (I):
HO(CH₂CH₂O)ₙ-R (I),
wherein R represents a phytosterol residue or a phytostanol residue, and n is a number of 5 to 100;
(2) a poorly water-soluble UV-absorbing agent; and
(3) water,
in the water-containing composition, to thereby enhance stability of the drug and/or dye, wherein the ratio by mass of the poorly water-soluble UV-absorbing agent to the polyoxyethylene addition compound (I) is 1 or higher.
